# EUROPÄISCHE PATENTANMELDUNG

(11) **EP 1 362 548 A1**
(43) Veröffentlichungstag der Anmeldung: **19.11.2003**
(21) Anmeldenummer: 03010355.0
(22) Anmeldetag: 08.05.2003
(51) Int. Cl.: A61B 3/12, A61B 3/14, A61B 5/00

(54) **Diagnoseeinrichtung zur Identifikation Schlaganfall-gefährdeter Patienten mittels Funduskamera**

(30) Priorität: 15.05.2002 DE 10221472
(71) Anmelder: e-EyeCare GmbH, 91052 Erlangen (DE)
(72) Erfinder: Michelson, Georg, Prof. Dr., 91083 Baiersdorf (DE)
(74) Vertreter: Gosdin, Michael

(57) **Zusammenfassung**

Die Erfindung betrifft eine Diagnoseeinrichtung (1) zur Diagnose von Gefäßerkrankungen eines Patienten. Die Vorrichtung weist auf: eine Kamera (2) zum Fotografieren des Hintergrundes mindestens eines Auges des Patienten; Mittel (3) zum digitalen Speichern des mit der Kamera (2) aufgenommenen Bildes des Augenhintergrundes; Mittel (4) zum Lesen des gespeicherten Bildes und Mittel (5) zur Bestimmung vorgegebener Parameter durch Auswertung des gespeicherten Bildes. Damit wird eine schnelle und kostengünstige Untersuchung möglich, um Schlaganfall-gefährdete Risikopatienten identifizieren zu können.

## Beschreibung

Die Erfindung betrifft eine Diagnoseeinrichtung zur Diagnose von Gefäßerkrankungen eines Patienten.

Patienten, die einen Schlaganfall erleiden, weisen in der Regel bereits lange vor dem Schlaganfall, meist mehrere Jahre davor, einen arteriellen Hypertonus auf. Daneben gibt es eine Anzahl bekannter Risikofaktoren, die das Risiko eines Schlaganfalls erhöhen (Übergewicht, etc.). Trotzdem war es bislang sehr schwierig, eine zutreffende Diagnose ohne aufwendige Untersuchungen vorzunehmen, die Auskunft darüber geben, wie hoch die aktuelle Gefahr eines Schlaganfalls ist.

Ein verbessertes Diagnostizierverfahren zur Beurteilung des Risikos eines Schlaganfalls ist von L. D. Hubbard et al. in "Methods for evaluation of retinal microvascular abnormalities associated with hypertension/sclerosis in the Atherosclerosis Risk in Communities Study", Ophthalmology, Vol. 106, 2269-2280, 1999, beschrieben. Dabei wird der Augenhintergrund und hier namentlich die Netzhaut betrachtet. Die Gefäße der Netzhaut und diejenigen des Gehirns sind sehr ähnlich. Beide Gefäße zeigen frühzeitig Veränderungen an, aufgrund derer auf erhöhtes Schlaganfall-Risiko geschlossen werden kann. Langandauernder Bluthochdruck verändert die Gefäße im Gehirn und in der Netzhaut in charakteristischer und messtechnisch erfassbarer Weise. Damit sind die Gefäße der Netzhaut sichtbare Indikatoren für Gefäßschäden im Gehirn, da die Netzhaut Teil des Gehirns ist.

Im Rahmen der erwähnten Diagnosemethode wird das Verhältnis zwischen der Summe aller arteriellen Gefäßdurchmesser und der Summe aller venösen Gefäßdurchmesser (a-v-ratio) ermittelt. Der Quotient liefert einen zuverlässigen Anhaltspunkt dafür, wie hoch das Risiko eines Schlaganfalls zu beurteilen ist.

Es würde vielen Menschen viel Leid ersparen und darüber hinaus auch die Kostenbelastung durch das Gesundheitswesen wesentlich verringern, wenn es möglich wird, im größeren Umfang und in besonders einfacher Weise die genannte Untersuchung durchzuführen, die eine leichte und zutreffende Diagnose ermöglicht, wie groß die Gefahr eines Schlaganfalls ist. Im gegebenen Falle könnte man Schlaganfall-gefährdete Patienten frühzeitig ausfindig machen und therapeutische Maßnahmen einleiten, bevor es zu einem Schlaganfall kommt; ein frühzeitiges Erkennen des Risikos und das frühzeitige Einleiten von Behandlungsmaßnahmen senken die Gefahr eines Schlaganfalls erheblich.

Voraussetzung hierfür ist eine einfache Untersuchungsmethode und die apparative Ausstattung dafür, eine solche Untersuchung in größerem Umfang und in kostengünstiger Weise durchführen zu können. Gerade hier hat das beschriebene Verfahren seine Nachteile: Zwar ist die Untersuchung selber nur mit wenig Aufwand verbunden; allerdings muss - um einen großen Nutzen aus dem Verfahren ziehen zu können - eine große Anzahl von Patienten untersucht werden, um die Risikopatienten zu identifizieren. Bislang bekannte Diagnosevorrichtungen sind jedoch nicht dazu geeignet, die benötigte Anzahl von Untersuchungen schnell und kostengünstig durchführen zu können.

Der Erfindung liegt daher die **Aufgabe** zugrunde, eine Diagnosevorrichtung zu schaffen, mit der es möglich ist, in sehr einfacher Weise die erforderlichen Daten zu erheben und auszuwerten, um in effizienter und damit kostengünstiger Art eine Vielzahl von Patienten untersuchen zu können. Die Vorrichtung soll es dabei insbesondere ermöglichen, die benötigen Informationen zur Diagnose den beteiligten Personen in einfacher Weise bereitzustellen und ansonsten die apparative Voraussetzung dafür zu schaffen, dass die Auswertung der Daten unterstützt wird. Die Diagnosevorrichtung soll einfach aufgebaut sein und eine schnelle und einfache Untersuchung ermöglichen. Sie soll somit die Voraussetzung dafür schaffen, eine Vielzahl von Patienten kostengünstig untersuchen und gefährdete Personen schnell identifizieren zu können.

Die **Lösung** dieser Aufgabe durch die Erfindung besteht in einer Diagnosevorrichtung, die aufweist:
- eine Kamera zum Fotografieren des Hintergrundes mindestens eines Auges des Patienten;
- Mittel zum digitalen Speichern des mit der Kamera aufgenommenen Bildes des Augenhintergrundes;
- Mittel zum Lesen des gespeicherten Bildes und
- Mittel zur Bestimmung vorgegebener Parameter durch Auswertung des gespeicherten Bildes.

Zur präzisen Aufnahme des Augenhintergrundes ist bevorzugt vorgesehen, dass die Vorrichtung eine Haltevorrichtung zum Halten und Positionieren des Kopfes des Patienten aufweist.

Mit Vorteil steht die Kamera mit einem Bildverarbeitungssystem in Verbindung, das das von der Kamera aufgenommene Bild des Augenhintergrundes in ein digital speicherbares Format transformiert. Dabei steht das Bildverarbeitungssystem mit dem Mittel zum digitalen Speichern des Bildes des Augenhintergrundes vorzugsweise über eine Datenfernübertragungseinrichtung in Verbindung. Gleichermaßen kann vorgesehen sein, dass das Mittel zum digitalen Speichern des Bildes des Augenhintergrundes über eine Datenfernübertragungseinrichtung mit dem Mittel zum Lesen des gespeicherten Bildes in Verbindung steht.

Besonders bevorzugt ist vorgesehen, dass einerseits die Kamera zum Fotografieren des Hintergrundes des Auges, andererseits die Mittel zum digitalen Speichern des mit der Kamera aufgenommenen Bildes und schließlich die Mittel zum Lesen des gespeicherten Bildes samt dem Mittel zur Bestimmung vorgegebener Parameter durch Auswertung des gespeicherten Bildes an drei voneinander weit beabstandeten Orten angeordnet sind, die jeweils über eine Datenfernübertragungseinrichtung miteinander kommunizieren. Als Kommunikationsmittel, also als Datenfernübertragungseinrichtung, kommt insbesondere das Internet zum Einsatz.

Die Kamera zum Fotografieren des Hintergrundes des Auges ist bevorzugt eine hochauflösende Digitalkamera. Dabei ist insbesondere an eine Non-Mydriatic-Funduskamera gedacht, die sich in besonders einfacher Weise zur Aufnahme des Augenhintergrundes eignet.

Wichtig ist die Sicherstellung, dass nur Berechtigte Zugriff auf das System und insbesondere auf die gespeicherten Daten haben. Daher ist nach einer Fortbildung vorgesehen, dass Mittel vorhanden sind, um insbesondere den unbefugten Zugriff auf die Mittel zum digitalen Speichern zu verhindern.

Hinsichtlich der Auswertemittel ist bevorzugt vorgesehen, dass diese zur Berechnung des Quotienten aus der Summe aller arteriellen Gefäßdurchmesser und der Summe aller venösen Gefäßdurchmesser, zumindest in einem ausgewählten Ausschnitt des aufgenommenen Hintergrundes des Auges, geeignet sind. Dafür kann eine Bildverarbeitungssoftware zum Einsatz kommen, die vorzugsweise so ausgeführt ist, dass die Auswertemittel zur automatischen Bilderkennung und -auswertung geeignet sind, um arterielle und venöse Gefäße zu erkennen und deren Durchmesser zu bestimmen.

Um eine effiziente Datenverwaltung zu ermöglichen, ist gemäß einer weiteren Fortbildung vorgesehen, dass die Mittel zum digitalen Speichern des mit der Kamera aufgenommenen Bildes des Augenhintergrundes dazu geeignet sind, zum Speicherbereich für das Bild einen Speicherbereich zuzuordnen, der einen ärztlichen Befund beinhaltet. Dabei kann der ärztliche Befund über Eingabemittel in den Speicherbereich für den Befund eingegeben werden.

Der erfindungsgemäße Vorschlag schafft eine Diagnosevorrichtung, die automatisch die Berechnung des Verhältnisses der Summe aller arteriellen Gefäßdurchmesser und der Summe aller venösen Gefäßdurchmesser, also das a-v-ratio, aus retinalen Fundusaufnahmen vornehmen kann, wodurch der Arzt, der die Diagnose stellt, in seiner Arbeit wesentlich erleichtert wird. Durch den Transfer der Informationen durch Datenleitungen vom Untersuchungsort (Ort der Kamera) zum Auswertezentrum (Ort der Auswertemittel) wird die benötigte Flexibilität geschaffen, um in kurzer Zeit eine große Anzahl von Patienten untersuchen zu können.

Es ist namentlich möglich, bei einer großen Anzahl Patienten anlässlich speziell vorgesehener Termine Aufnahmen vom Augenhintergrund anzufertigen und die Daten später und an einem anderen Ort (im Auswertezentrum) synergetisch zu analysieren. Damit wird eine ökonomische Ermittlung von Patienten mit Schlaganfallgefährdung möglich.

Die vorgeschlagene Diagnosevorrichtung erlaubt also eine einrichtungsübergreifende, evidenzbasierte Kommunikation zur Früherkennung von Schlaganfall-gefährdeten Patienten anhand der Netzhautgefäße und eine elektronische Zur-Verfügung-Stellung der benötigten Information an autorisierte Ärzte und Patienten.

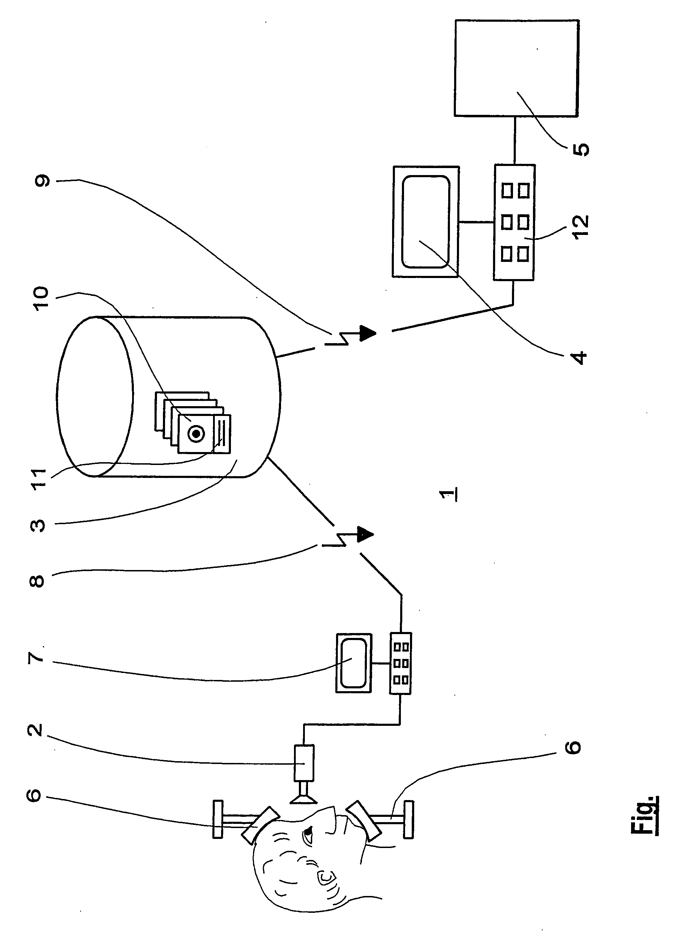

In der Zeichnung ist ein Ausführungsbeispiel der Erfindung dargestellt. Die einzige Figur zeigt sehr schematisch die komplette Diagnoseeinrichtung zur Identifikation Schlaganfall-gefährdeter Patienten.

Die gesamte Diagnoseeinrichtung ist mit der Bezugsziffer 1 bezeichnet. Sie weist zunächst - am Untersuchungsort des Patienten - eine Haltevorrichtung 6 für den Kopf des Patienten auf. Der Kopf wird auf die Haltevorrichtung 6 aufgelegt und ruhig gehalten. Dann wird mit einer Non-Mydriatic-Funduskamera 2 der Hintergrund des Auges des Patienten fotografiert. Die Kamera 2 ist mit einem Bildverarbeitungssystem 7 verbunden, das das fotografierte Bild in eine digital speicherbare Form bringt, sofern die Kamera 2 nicht direkt ein Digitalbild liefert. Über eine geschützte Datenfernübertragungseinrichtung 8, die nur sehr schematisch skizziert ist, wird das digitale Bild des Augenhintergrundes zu einem digitalen Speichermittel 3 geleitet und dort hinterlegt. Im Speichermittel 3 befindet sich eine Vielzahl von Files, wobei jedes File eine Patientenakte darstellt. Das File eines Patienten hat einen Speicherbereich 10 für das digitalisierte Bild des Augenhintergrundes. Ferner ist dort - unter anderem - ein Speicherbereich 11 vorhanden, in dem der ärztliche Befund gespeichert werden kann.

Die digitalen Speichermittel 3 sind über eine Datenfernübertragungseinrichtung 9, die wieder nur sehr schematisch skizziert ist, mit einem Lesemittel 4 verbunden, das die Fotografie des Augenhintergrundes wieder sichtbar machen kann. Im einfachsten Falle handelt es sich um einen Monitor, auf dem sich der Arzt den Augenhintergrund anzeigen lassen kann. Mit dem Lesemittel 4 ist ein Auswertemittel 5 verbunden, das dazu in der Lage ist, über eine geeignete und an sich hinlänglich bekannte Bildauswertesoftware eine graphische Analyse des aufgenommenen Bildes des Augenhintergrundes durchführen zu können. Hierbei wird mittels geeigneter digitaler Verfahren in einem vorgegebenen Abschnitt der Fotografie eine Ermittlung des Durchmessers (bzw. des auf der Fotografie sichtbaren Querschnitts) der arteriellen Gefäße der Netzhaut einerseits und der venösen Gefäße andererseits vorgenommen. Die Auswertemittel bilden von den ermittelten Volumina (bzw. Flächen) die Summe und anschließend den Quotienten der Summen. Der genannte Quotient kann nach automatischer Segmentierung der retinalen Arterien und Venen und automatischer Bestimmung der Durchmesser aller Gefäße größer als 30 µm berechnet werden.

Dieser Quotient - das a-v-ratio - aus der retinalen Fundusaufnahme liefert nach L. D. Hubbard (s. o.) ein Kriterium für die Beurteilung des Schlaganfallrisikos, dem der Patient ausgesetzt ist. Damit erfolgt eine zahlenmäßige Beurteilung des Zustands der Augenhintergrundgefäße hinsichtlich Gefäßschäden durch Bluthochdruck.

Der Arzt kann daher nach automatischer Ermittlung des a-v-ratio durch die Auswertemittel 5 seine Diagnose erstellen und diese über die Eingabemittel 12 und die Datenfernübertragungseinrichtung 9 im Speicherbereich 11 hinterlegen.

Der Ablauf der Untersuchung kann folgendermaßen erfolgen:

Zunächst wird die Bevölkerung über die Untersuchungsmethode informiert und an einer Untersuchung Interessierte zum Untersuchungsort einbestellt. Am Untersuchungsort werden die benötigten persönlichen Daten vom Patienten aufgenommen, um ihn einerseits identifizieren zu können und - für die spätere Diagnose - dem Arzt auch die Informationen an die Hand zu geben, die für die Diagnose erforderlich sind (Alter, Geschlecht, Gewicht, Größe, etc.). Gleichermaßen werden versicherungsspezifische Daten erhoben, beispielsweise durch direktes Lesen der Versicherten-Chipkarte in einem entsprechenden Lesegerät. Dann wird nach Erheben einer Kurzanamnese mit der Kamera 2 der Augenhintergrund fotografiert. Dies kann durch eine Medizinisch Technische Assistentin ohne Augentropfen und ohne Mydriasis erfolgen. Die Fotografie des Augenhintergrundes erfolgt gänzlich kontaktlos, so dass keinerlei Belastung des Patienten auftritt. Über die Bildverarbeitungsmittel 7 wird der Datensatz des Bildes zusammen mit den erhobenen Patententendaten zum Speichermittel 3 geleitet.

Im Auswertezentrum ruft sich der diagnostizierende Arzt den Fall, d. h. die gespeicherten Daten, über die Lesemittel 4 aus dem Speichermittel 3 auf. Es erfolgt in den Auswertemitteln 5 eine Auswertung des Fundusbildes durch spezielle Bildverarbeitungssoftware und eine anschließende Berechnung des genannten a-v-ratio (Quotient). Der Arzt kann dann die Diagnose erstellen und über die Eingabemittel 12 diese im Speicherbereich 11 der Speichermittel 3 abspeichern.

Über (geschützten) Internetzugang mittels Passwort ist es anschließend von einem beliebigen Ort aus möglich, auf das Speichermittel 3 und einen speziellen Patientenfall zuzugreifen und das Ergebnis dem Patienten mitzuteilen.

Für den Datenschutz kommen übliche und an sich bekannte Elemente zum Einsatz. Jeder Arzt ist identifiziert über eine Chipkarte mit Attribut. Die Bewegung der Daten erfolgt gesichert und nachvollziehbar, indem jede Transaktion protokolliert wird und damit die Authentizität der Befunde jederzeit nachprüfbar ist. Der Datentransfer wird dadurch gesichert, dass die Daten zwischen den einzelnen Stationen (zwischen Bildverarbeitungssystem 7 und Speichermittel 3 bzw. zwischen Speichermittel 3 und Lesemittel 4) verschlüsselt übertragen werden (128-Bit-Schlüssel). Sensible Daten können im Speichermittel 3 (Server) verschlüsselt hinterlegt werden. Durch eine Verschlüsselung werden keine personenbezogenen Daten gespeichert. Zur Datensicherung ist vorgesehen, dass eine Speicherung stets auf zwei Festplatten gleichzeitig erfolgt. Der Server (Speichermittel 3) kann physikalisch durch ein elektronisches Schließsystem im Sicherheits-Rechenzentrum gesichert werden. Ferner kann der Server über eine Fire-Wall gesichert werden.

Zu bemerken ist noch folgendes: Die gesamte Untersuchung einschließlich Diagnose erfolgt weitgehend aufgrund physikalisch messbarer Parameter (quantitative Analyse der Gefäßveränderungen); für die Beurteilung des Schlaganfall-Risikos werden vorgegebene Quotienten berechnet, wobei hierzu wiederum auf eine Auswertung von bildbezogenen Daten zurückgegriffen wird (Bestimmung der arteriellen und venösen Gefäßdurchmesser größer 30 µm, Aufsummation, Quotientenbildung). Daher ist es nicht unbedingt erforderlich, dass überhaupt ärztliche Hilfe bei der Erstellung eines Befundes in Anspruch genommen werden muss. Vielmehr ist es auch genauso denkbar, dass die erläuterte Vorgehensweise alleine von Personen durchgeführt wird, die keine ärztliche Ausbildung haben, sondern lediglich die Datenhandhabung und automatische Auswertung der Daten im Auswertemittel betreuen; letztlich kann die gesamte Untersuchung auch weitgehend automatisiert und maschinengestützt erfolgen, was die Effizienz des Systems weiter erhöht.

Hierbei ist die Möglichkeit der Fernbefundung (am Ort der Lesemittel 4 und Auswertemittel 5) - mit und ohne Arzt - ein wichtiges Instrument, um eine schnelle und preiswerte Untersuchung einer großen Anzahl von Patienten möglich zu machen.

### Bezugszeichenliste:

- 1: Diagnoseeinrichtung
- 2: Kamera
- 3: digitale Speichermittel
- 4: Lesemittel
- 5: Auswertemittel
- 6: Haltevorrichtung
- 7: Bildverarbeitungssystem
- 8: Datenfernübertragungseinrichtung
- 9: Datenfernübertragungseinrichtung
- 10: Speicherbereich für das Bild
- 11: Speicherbereich für den ärztlichen Befund
- 12: Eingabemittel

## Patentansprüche

1. Diagnoseeinrichtung (1) zur Diagnose von Gefäßerkrankungen eines Patienten, die aufweist:
- eine Kamera (2) zum Fotografieren des Hintergrundes mindestens eines Auges des Patienten;
- Mittel (3) zum digitalen Speichern des mit der Kamera (2) aufgenommenen Bildes des Augenhintergrundes;
- Mittel (4) zum Lesen des gespeicherten Bildes und
- Mittel (5) zur Bestimmung vorgegebener Parameter durch Auswertung des gespeicherten Bildes.

2. Diagnoseeinrichtung nach Anspruch 1, **dadurch gekennzeichnet, dass** sie eine Haltevorrichtung (6) zum Halten und Positionieren des Kopfes des Patienten aufweist.

3. Diagnoseeinrichtung nach Anspruch 1 oder 2, **dadurch gekennzeichnet, dass** die Kamera (2) mit einem Bildverarbeitungssystem (7) in Verbindung steht, das das von der Kamera (2) aufgenommene Bild des Augenhintergrundes in ein digital speicherbares Format transformiert.

4. Diagnoseeinrichtung nach Anspruch 3, **dadurch gekennzeichnet, dass** das Bildverarbeitungssystem (7) mit dem Mittel (4) zum digitalen Speichern des Bildes des Augenhintergrundes über eine Datenfernübertragungseinrichtung (8) in Verbindung steht.

5. Diagnoseeinrichtung nach einem der Ansprüche 1 bis 4, **dadurch gekennzeichnet, dass** das Mittel (4) zum digitalen Speichern des Bildes des Augenhintergrundes über eine Datenfernübertragungseinrichtung (9) mit dem Mittel (5) zum Lesen des gespeicherten Bildes in Verbindung steht.

6. Diagnoseeinrichtung nach einem der Ansprüche 1 bis 5, **dadurch gekennzeichnet, dass** einerseits die Kamera (2) zum Fotografieren des Hintergrundes des Auges, andererseits die Mittel (3) zum digitalen Speichern des mit der Kamera (2) aufgenommenen Bildes und schließlich die Mittel (4) zum Lesen des gespeicherten Bildes samt dem Mittel (5) zur Bestimmung vorgegebener Parameter durch Auswertung des gespeicherten Bildes an drei voneinander weit beabstandeten Orten angeordnet sind, die jeweils über eine Datenfernübertragungseinrichtung (8, 9) miteinander kommunizieren.

7. Diagnoseeinrichtung nach einem der Ansprüche 4 bis 6, **dadurch gekennzeichnet, dass** die Datenfernübertragungseinrichtung (8, 9) das Internet ist.

8. Diagnoseeinrichtung nach einem der Ansprüche 1 bis 7, **dadurch gekennzeichnet, dass** die Kamera (2) zum Fotografieren des Hintergrundes des Auges eine hochauflösende Digitalkamera ist.

9. Diagnoseeinrichtung nach einem der Ansprüche 1 bis 8, **dadurch gekennzeichnet, dass** die Kamera (2) zum Fotografieren des Hintergrundes des Auges eine Non-Mydriatic-Funduskamera ist.

10. Diagnoseeinrichtung nach einem der Ansprüche 1 bis 9, **dadurch gekennzeichnet, dass** Mittel vorhanden sind, um den unbefugten Zugriff auf die Mittel (3) zum digitalen Speichern zu verhindern.

11. Diagnoseeinrichtung nach einem der Ansprüche 1 bis 10, **dadurch gekennzeichnet, dass** die Mittel (5) zur Bestimmung vorgegebener Parameter zur Berechnung des Quotienten aus der Summe aller arteriellen Gefäßdurchmesser und der Summe aller venösen Gefäßdurchmesser, zumindest in einem Ausschnitt des aufgenommenen Hintergrundes des Auges, geeignet sind.

12. Diagnoseeinrichtung nach Anspruch 11, **dadurch gekennzeichnet, dass** die Mittel (5) zur Bestimmung vorgegebener Parameter zur automatischen Bilderkennung und -auswertung geeignet sind, um arterielle und venöse Gefäße zu erkennen und deren Durchmesser zu bestimmen.

13. Diagnoseeinrichtung nach einem der Ansprüche 1 bis 12, **dadurch gekennzeichnet, dass** die Mittel (3) zum digitalen Speichern des mit der Kamera (2) aufgenommenen Bildes des Augenhintergrundes dazu geeignet sind, zum Speicherbereich für das Bild (10) einen Speicherbereich (11) zuzuordnen, der einen ärztlichen Befund beinhaltet.

14. Diagnoseeinrichtung nach Anspruch 13, **dadurch gekennzeichnet, dass** über Eingabemittel (12) der ärztliche Befund in den Speicherbereich (11) für den ärztlichen Befund eingegeben werden kann.
